(19) Europäisches Patentamt
European Patent Office
Office européen des brevets

(11) Veröffentlichungsnummer: **0 464 430 A1**

(12) **EUROPÄISCHE PATENTANMELDUNG**

(21) Anmeldenummer: **91109826.7**

(22) Anmeldetag: **15.06.91**

(51) Int. Cl.[5]: **C07D 207/335**, C07D 207/337, A61K 31/40

(30) Priorität: **29.06.90 DE 4020851**

(43) Veröffentlichungstag der Anmeldung: **08.01.92 Patentblatt 92/02**

(84) Benannte Vertragsstaaten: **AT BE CH DE DK ES FR GB GR IT LI NL SE**

(71) Anmelder: **CASSELLA Aktiengesellschaft**
**Hanauer Landstrasse 526**
**W-6000 Frankturt am Main 60(DE)**

(72) Erfinder: **Zoller, Gerhard, Dr.**
**Höhenstrasse 8**
**W-6369 Schöneck(DE)**
Erfinder: **Schindler, Ursula, Dr.**
**Robert-Stolz-Strasse 56**
**W-6232 Bad Soden(DE)**

(74) Vertreter: **Urbach, Hans-Georg, Dr. et al**
**CASSELLA AKTIENGESELLSCHAFT**
**Patentabteilung Hanauer Landstrasse 526**
**W-6000 Frankturt am Main 60(DE)**

# (54) 2-(Aminoalkyl)-pyrrolaldehyde, Verfahren zu ihrer Herstellung und ihre Verwendung.

(57) 2-(Aminoalkyl)-pyrrolaldehyde der allgemeinen Formel I

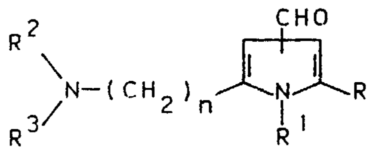

(I)

worin
R: Wasserstoff, Alkyl;
$R^1$: z.B. Wasserstoff, Alkyl, Phenyl;
$R^2$, $R^3$: z.B. Wasserstoff, Alkyl, Alkanoyl bedeuten, besitzen wertvolle pharmakologische Eigenschaften.

EP 0 464 430 A1

Untersuchungen haben gezeigt, daß sowohl die chemische Stabilität als auch die pharmakologischen Aktivitäten der in der Europäischen Patentschrift EP 0 124 067 beschriebenen 2-(Aminoalkyl)-pyrrol-derivate durch die Einführung von Aldehydgruppen deutlich gesteigert werden können.

Die Erfindung betrifft 2-(Aminoalkyl)-pyrrolaldehyde der allgemeinen Formel I

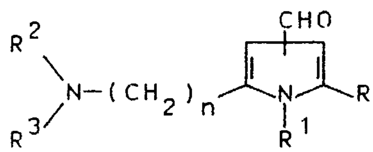

(I)

worin

R: Wasserstoff, Alkyl($C_1$-$C_3$);

$R^1$: Wasserstoff, Alkyl($C_1$-$C_5$), Cyanoalkyl($C_1$-$C_4$), Alkoxy($C_1$-$C_4$)carbonyl, Hydroxycarbonylalkyl($C_1$-$C_4$), Alkoxy($C_1$-$C_4$)carbonylalkyl($C_1$-$C_4$), $R^4$($R^5$)N-carbonylalkyl($C_1$-$C_4$), Alkyl($C_1$-$C_6$)carbonylaminoalkyl($C_1$-$C_4$), Phenyl-carbonylaminoalkyl($C_1$-$C_4$), Phenoxy-alkyl($C_1$-$C_3$)-carbonylaminoalkyl($C_1$-$C_4$), Phenyl, Phenylalkyl-($C_1$-$C_4$,); $R^2$, $R^3$: unabhängig voneinander Wasserstoff, Alkyl($C_1$-$C_6$),Alkanoyl($C_1$-$C_6$), Phenylalkyl($C_1$-$C_4$)-carbonyl, Phenoxy-alkyl($C_1$-$C_3$)carbonyl, Benzoyl, Pyridylcarbonyl, oder zusammen mit dem Stickstoffatom, an das sie gebunden sind, einen 5-gliedrigen Ring bilden;

$R^4$, $R^5$: unabhängig voneinander Wasserstoff, Alkyl($C_1$-$C_4$) oder zusammen mit dem Stickstoffatom, an das sie gebunden sind, einen 5-gliedrigen Ring bilden; wobei die Phenyl-, Phenoxy- oder Benzoylsubstituenten auch durch Halogen, Alkyl($C_1$-$C_4$), Hydroxy, Alkoxy($C_1$-$C_4$), $R^4$($R^5$)N, Mercapto, Alkylmercapto($C_1$-$C_4$), Nitro, Cyano, Hydroxycarbonyl, Alkoxy($C_1$-$C_4$)carbonyl, Alkoxy($C_1$-$C_4$)carbonylalkyl($C_1$-$C_4$), Formyl, Alkanoyl($C_1$-$C_4$), ein- oder mehrfach substituiert sein können;

n: 1, 2 oder 3;

bedeuten,

sowie ihre pharmakologisch annehmbaren Säureadditionssalze bei erfindungsgemäßen Verbindungen, die eine basische Gruppe enthalten.

Die Erfindung betrifft auch Verfahren zur Herstellung der Verbindungen I sowie ihre Verwendung als Arzneimittel.

Die Alkyl-, Alkoxy-, Alkanoyl-, Alkanoyloxy-, Alkylen-, Alkoxycarbonylreste können, auch wenn sie in Verbindung mit anderen Resten auftreten, geradkettig oder verzweigt sein. Für Phenylalkyl kommt vor allem Phenethyl, vorzugsweise Benzyl, in Betracht.

In den für $R^1$ bzw. $R^2$ und $R^3$ stehenden Resten: Phenylcarbonylaminoalkyl($C_1$-$C_4$), Phenyl, Phenylalkyl-($C_1$-$C_4$), Phenoxy-alkyl($C_1$-$C_3$)carbonylaminoalkyl($C_1$-$C_4$), Phenylalkyl($C_1$-$C_4$)carbonyl, Phenoxy-alkyl($C_1$-$C_3$)carbonyl, Benzoyl können die Phenyl-, Phenoxy- und Benzoyl-Reste ein oder mehrfach, beispielsweise ein-, zwei- oder dreifach, vorzugsweise ein- oder zweifach, mit den vorstehend genannten Substituenten substituiert sein.

Als Halogensubstituenten der Phenyl-, Phenoxy- oder Benzoylreste sind z. B. Fluor, Jod, vor allem Brom und vorzugsweise Chlor zu nennen.

Die für R stehenden Alkylreste können Methyl, Ethyl, Propyl und Isopropyl sein. Für den Substituenten R ist Methyl bevorzugt.

Beispiele für Substituenten, die für $R^1$ stehen können, sind: Wasserstoff, Methyl, Ethyl, Propyl, Pentyl, Isopropyl, Butyl(1), Butyl(2), Isobutyl, tert.Butyl, Isopentyl, Cyanomethyl, 2-Cyanoethyl, 3-Cyano-propyl, Methoxycarbonyl, n-Propoxy-carbonyl, i-Propoxy-carbonyl, n-Butoxy-carbonyl t-Butoxy-carbonyl, Benzyloxycarbonyl, Methoxycarbonylmethyl, n-Propoxy-carbonylmethyl, i-Butoxycarbonylmethyl, 2-(Methoxycarbonyl)-ethyl, 2-(Ethoxycarbonyl)-ethyl, 2-(i-Propoxycarbonyl)-ethyl, 2-(Methoxycarbonyl)-propyl, 2-(Ethoxycarbonyl)-propyl, Aminocarbonylmethyl, 2-(Aminocarbonyl)-ethyl, N,N-Dimethylaminocarbonylmethyl, Ethylaminocarbonylethyl, Pyrrolidino-carbonyl-methyl, N,N-Dimethylaminocarbonyl-ethyl, Phenyl, 2-Methylphenyl, 3-Methylphenyl, 4-Methylphenyl, 2-Methoxyphenyl, 3-Methoxyphenyl, 4-Methoxyphenyl, 3,4-Dimethoxyphenyl, o-, m-, p-Chlorphenyl, 4-Dimethylaminophenyl, 4-Mercaptophenyl, Benzyl, 4-Methoxybenzyl, 1-(Methoxy-carbonyl)-2-mercapto-phenyl, 2-(Acetamino)-ethyl, 3-(Acetamino)-propyl, 2-,3-oder 4-(Ethylcarbonylamino)-butyl, 2-, 3- oder 4-(Propylcarbonylamino)-butyl, Methoxycarbonylmethyl, 1- oder 2-Methoxycarbonylethyl, 1- oder 2-Ethoxycarbonylethyl, 1- oder 2-Propoxycarbonyl-ethyl.

Als Substituenten $R^1$ sind bevorzugt: Wasserstoff, Alkyl($C_1$-$C_6$)carbonylaminoalkyl($C_1$-$C_4$), insbesondere 2-Acetamino-ethyl, Alkoxy($C_1$-$C_4$)-carbonylalkyl($C_1$-$C_4$), insbesondere Methoxy-carbonyl-methyl.

Für $R^2$ und $R^3$ können beispielsweise auch unabhängig voneinander stehen: Wasserstoff, Methyl, Ethyl,

Propyl, Isopropyl, Butyl(1), Butyl(2), Isobutyl, Isopentyl, Pentyl, Formyl, Acetyl, Propionyl, Butyryl, Isobutyryl, Valeryl, Isovaleryl, Pivaloyl, Benzoyl, o-, m-, p-Methylbenzoyl, 2,4-Dimethylbenzoyl, o-, m-, p-Methoxybenzoyl, 2,4-Dimethoxybenzoyl, 3,4-Dimethoxybenzoyl, o-, m-, p-Chlorbenzoyl, o-, m-, p-Brombenzoyl, o-, m-, p-Aminobenzoyl, o-, m-, p-Hydroxybenzoyl, 4-Hydroxy-3-methoxybenzoyl, 2-Phenyl-acetyl, 2-, 3-, 4-Pyridyl-carbonyl, 2-, 3- oder 4-Nitrobenzoyl, 2-, 3-oder 4-Cyanobenzoyl, 2-, 3-oder 4-Alkoxy($C_1$-$C_4$)-carbonyl-benzoyl, 2-, 3- oder 4-Ethoxy-carbonyl-benzoyl, 2-, 3- oder 4-Propoxy-carbonyl-benzoyl, 2-, 3- oder 4-sec-Butoxy-carbonyl-benzoyl.

Beispiele für 5-gliedrige Ringe, die $R^2$ und $R^3$, sowie $R^4$ und $R^5$, zusammen mit dem Stickstoffatom, an das sie gebunden sind, bilden können, sind Pyrrolidin-2-on-1-yl und Phthalimidyl.

Bevorzugte Substituenten für $R^2$ sind: Alkanoyl($C_1$-$C_6$),insbesondere Acetyl, Benzoyl, Phenylalkyl($C_1$-$C_4$)carbonyl, insbesondere Benzylcarbonyl, Phenoxy-alkyl($C_1$-$C_3$)carbonyl, insbesondere Phenoxy-methyl-carbonyl, wobei die Phenylgruppe des Benzoyls oder Phenalkylcarbonyls, insbesondere Benzylcarbonyls oder die Phenoxygruppe des Phenoxy-alkyl($C_1$-$C_3$))carbonyls, insbesondere des Phenoxymethyl-carbonyls, vorzugsweise auch einen oder zwei Substituenten, insbesondere Halogen, Nitro und/oder Alkoxy($C_1$-$C_4$) tragen kann. Als derartige Substituenten kommen insbesondere Chlor, Methoxy und/oder Nitro in Betracht. Für $R^2$ sind besonders bevorzugt: Acetyl, Chlorphenoxyacetyl, insbesondere 4-Chlor-phenoxyacetyl, Nitrobenzoyl, insbesondere 4-Nitrobenzoyl und Dimethoxybenzoyl, insbesondere 3,4-Dimethoxybenzoyl.

Für $R^3$ ist Wasserstoff und für n ist 1 bevorzugt.

In vielen Fallen zeigen die erfindungsgemäßen Verbindungen, bei denen die Aldehydgruppe in 4-Stellung des Pyrrolkernes steht eine höhere pharmakologische Wirksamkeit als wenn sie in 3-Stellung des Pyrrolringes steht.

Bevorzugte Verbindungen der Formel I sind solche, die eine oder insbesondere mehrere Bedeutungen für R, $R^1$, $R^2$, $R^3$ und n besitzen. Besonders bevorzugte erfindungsgemäße Verbindungen sind: Essigsäure-N-((1-(2-acetylaminoethyl)-4-formyl-5-methyl-1H-pyrrol-2-yl)methyl)-amid, 1H-Pyrrol-1-essigsäure-(2-(acetylaminoethyl)-4-formyl-5-methylester und 4-Chlorphenoxyessigsäure-N-((1-(2-acetylaminoethyl-3-formyl-5-methyl-1H-pyrrol-2-yl)methyl)-amid.

Die erfindungsgemäßen 2-(Aminoalkyl)-pyrrolaldehyde der allgemeinen Formel I werden dadurch hergestellt, daß ein 2-(Aminoalkyl)-pyrrol der Formel II

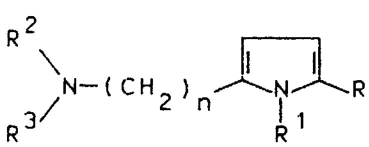

(II)

wobei R, $R^1$, $R^2$, $R^3$ und n die bereits genannten Bedeutungen besitzen, in an sich bekannter Weise durch Umsetzung mit einem Formylierungsreagenz formyliert wird. Für die Formylierung eignen sich viele in der Literatur beschriebene Methoden, vgl. z.B. Houben-Weyl, Methoden der Organischen Chemie, 4.Aufl., E3, S. 16 ff (1983). In speziellen Fällen ist die Variante nach Reimer-Tiemann durch Umsetzung der Pyrrole mit Chloroform im alkalischen Medium angebracht, bessere Ausbeuten ergibt jedoch häufig die Umsetzung der Pyrrole mit 1,1-Dihalogenethern und Friedel-Crafts-Katalysatoren (J. Med. Chem. 15, 97 (1972)) oder mit Trialkoxymethanen und Trifluoressigsäure (J. Org. Chem. 43 283 (1978)). Am einfachsten führt die Vilsmeier-Synthese, auch Vilsmeier-Haack-Reaktion genannt, durch Umsetzung der Pyrrole der allgemeinen Formel II mit einem Formamid und mit einem Komplexbildner bzw. Friedel-Crats-Katalysator zu den erfindungsgemäßen Verbindungen (Methodicum Chimicum 5 S. 234 (1975), J. Org. Chem. 28 3052 (1963)), die daher zur Herstellung der erfindungsgemäßen Verbindungen bevorzugt ist.

Geeignete Formamide sind z. B. N-Methyl-formanilid, N,N-disubstituierte Formamide, wie N-Formyl-morpholin und -piperidin, insbesondere aber N,N-Dimethylformamid. Als Komplexbildner wird vor allem Phosphoroxychlorid verwendet.

Phosphoroxychlorid kann durch andere Verbindungen wie z.B. Oxalylchlorid, Thionylchlorid, Sulfurychlorid, Phosgen, Di- oder Triphosgen, Cyanurchlorid oder Säurechloride, wie Acetyl- oder Benzoylchlorid ersetzt werden. Anstelle eines Formamids und eines Komplexbildners, wie Phosphoroxychlorid, kann auch ein als Vilsmeier-Reagenz bezeichnetes Halogenmethylendimethylammoniumhalogenid eingesetzt werden. Vorteilhaft wird die Reaktion in einem Lösungsmittel, z.B. einem Amid, wie Dimethylformamid, einem Aromaten, wie Toluol, halogenierten Kohlenwasserstoff wie 1,2-Dichlorethan, Methylen-chlorid, Chlorbenzolen, einem Ether, wie Tetrahydrofuran oder Ethylenglykoldimethylether, einem Ester wie Essigsäureethyl- oder -butylester, Nitrobenzol, oder einem Gemisch verschiedener Lösungsmittel durchgeführt. Auch Isonitrile in saurer Lösung eignen sich als Formylierungsagenzien für die Herstellung der erfindungsgemäßen

Verbindungen (Chem. Ber.94, 298 (1961)). Eine weitere Möglichkeit zur Formylierung der 2-(Aminoalkyl)-pyrrolderivate der Formel II ist die Umsetzung mit s-Triazin in Gegenwart von Chlorwasserstoff (Arch. Pharm. 302 828). Die zur Herstellung benötigten Ausgangsverbindungen der Formel II sind z.B. aus der EP-A1-0124067 oder USP 4 785 010 bekannt und können nach den dort beschriebenen Verfahren hergestellt werden. Bei der Formylierung der 2-(Aminoalkyl)-pyrrol-derivate der Formel II entstehen in der Regel Isomerengemische mit der Formylgruppe in 3-und 4-Stellung des Pyrrolrings, die durch bekannte Trennmethoden, wie fraktionierte Kristallisation oder Säulenchromatographie, getrennt werden können.

Sofern die erfindungsgemäßen Verbindungen der allgemeinen Formel I basische Reste enthalten, bilden sie mit anorganischen oder organischen Säuren Säureadditionssalze. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Äpfel-, Sulfamin-, Phenylpropion-, Glucon-, Ascorbin-, Nicotin-, Isonicotin-, Methansulfon-, p-Toluolsulfon-, Zitronen- oder Adipin-säure. Pharmakologisch annehmbare Säureadditionssalze werden bevorzugt. Die Säureadditionssalze werden wie üblich durch Vereinigung der Komponenten, zweckmäßigerweise in einem geeigneten Löse- oder Verdünnungsmittel, hergestellt.

Bei der Synthese der Verbindungen der Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, nach anschließendem Extrahieren gewonnen werden.

Die erfindungsgemäßen Verbindungen der allgemeinen Formel I und ihre pharmakologisch akzeptablen Säureadditionssalze besitzen wertvolle pharmakologische Eigenschaften. Sie sind zentral wirksam, beispielsweise zeigen sie encephalotrope und nootrope Wirkungen und dienen zur Behandlung von Krankheiten, die durch eine Einschränkung der Gehirnfunktionen bedingt sind, wie cerebraler Insuffizienz, cerebraler Alterungsvorgänge, verminderter Gedächtnisleistung, wie sie auch bei der Alzheimer Krankheit oder der Multi-Infarkt-Demenz auftreten. Sie zeigen eine ausgezeichnete Wirksamkeit in verschiedenen pharmakologischen Tests, wie z.B. bei der Verlängerung der Überlebenszeit unter Natriumnitrit-Hypoxie nach Gibson und Blass (J. Neurochemistry 27, 37 (1976)) oder bei durch Natriumnitrit induzierten Verhaltensveränderungen nach Peterson und Gibson (J. Pharmacol. exp. Ther. 222, 576 (1982)).

Auch in Tests, die direkt auf die Erfassung der Lern-und Gedächtnisleistung abzielen, sind die erfindungsgemäßen Verbindungen gut wirksam. In Lern- und Gedächtnistests, bei denen die Amnesie oder Ischämie experimentell induziet sind, wie z.B. bei der Scopolaminamnesie oder einer cerebralen Ischämie, zeigen die Verbindungen gute Wirkungen.

Die Verbindungen der allgmeinen Formel I und ihre physiologisch verträglichen Salze stellen somit eine Bereicherung der Pharmazie dar.

Die erfindungsgemäßen Verbindungen der allgemeinen Formel I und ihre pharmazeutisch akzeptablen Säureadditionssalze können daher am Menschen als Heilmittel z.B. bei der Behandlung oder Vorbeugung von Erkrankungen, die durch eine Einschränkung der Gehirnfunktion bedingt sind und bei der Behandlung oder Vorbeugung von cerebralen Alterungsvorgängen Anwendung finden.

Die Verbindungen der allgemeinen Formel I und ihre pharmazeutisch akzeptablen Säureadditionssalze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgmeinen Formel I oder eines Säureadditionssalzes davon, neben üblichen pharmazeutischen einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole,

Glycerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Isosorbidmononitrat, Glycerolnitrat, Molsidomin und Verapamil, $\beta$-Blocker, wie Propranolol, Oxprenolol, Atenolol, Metoprolol und Penbutolol. Darüberhinaus lassen sich die Verbindungen mit anderen nootrop wirksamen Substanzen, wie z.B. Piracetam, oder ZNSaktiven Substanzen, wie Pirlindol, Sulpirid etc. kombinieren.

Die Dosierung kann innerhalb weiter Grenzen variieren und ist in jedem einzelnen Fall den individuellen Gegebenenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,1 bis 50 mg, vorzugsweise 0,5 bis 10 mg Wirkstoff der Formel I oder eines pharmakologisch annehmbaren Salzes davon pro Dosis.

Die folgenden Beispiele 1 bis 8 betreffen die Herstellung von Verbindungen der Formel I, die Beispiele A bis H betreffen die Herstellung von Zubereitungen der Verbindungen der Formel I.

## Beispiel 1

4-Methoxybenzoesäure-N-((1-(2-acetylamino)ethyl)-(3)4-formyl-5-methyl-1H-pyrrol-2-yl)methyl)-amid

Zu 1,2 ml (15,5 mmol) wasserfreiem Dimethylformamid werden bei 15-20°C 1,4 ml (15 mmol) Phosphoroxychlorid in 5 ml 1,2-Dichlorethan innerhalb 10 Minuten zugetropft. Man rührt 10 Minuten nach und tropft dann 4,7 g 4-Methoxybenzoesäure-N-(1-(2-acetylamino)ethyl-5-methyl-1H-pyrrol-2-yl)methyl)-amid in 40 ml 1,2-Dichlorethan bei 15 - 20°C zu. Man rührt 3 h bei Raumtemperatur, hydrolysiert mit 2,7 g Natriumhydroxid, in 50 ml Wasser gelöst, versetzt mit Methylenchlorid und trennt die Phasen. Die Wasserphase wird mit Methylenchlorid extrahiert und die vereinigten organischen Phasen werden getrocknet und eingeengt. Der Rückstand wird über eine Kieselgelsäule mit Methylenchlorid als Laufmittel gereingt.

1. Verbindung:
4-Methoxybenzoesäure-N-((1-(2-acetylaminoethyl)-3-formyl -5-methyl-1H-pyrrol-2-yl)methyl)-amid
Schmelzpunkt: 155-160°C
1H-NMR (DMSO, TMS) $\delta$(ppm)
1,85 (s,3H); 2,25 (s, 3H); 3,3(m, 2H); 3,8 (s, 3H); 4,1 (m, 2H); 4,75 (d, 2H); 6,2 (s, 1H); 6,95 (d, 2H); 7,9 (d, 2H); 8,15 (m, 1H); 8,8 (m, 1H); 9,85 (s, 1H);

2. Verbindung:
4-Methoxybenzoesäure-N-((1-2-acetylaminoethyl)-4-formyl-5-methyl-1H-pyrrol-2-yl)methyl)-amid
Schmelzpunkt: 164-165°C
1H-NMR (DMSO, TMS), $\delta$(ppm);
1,85 (s, 3H); 2,5 (s, 3H); 3,3 (m, 2H); 3,8 (s, 3H); 4,0 (m, 2H); 4,5 (d, 2H); 6,3 (s, 1H); 7,0 (d, 2H); 7,9 (d, 2H); 8,2 (m, 1H); 8,75 (m, 1H); 9,75 (s, 1H).

## Beispiel 2

3,4-Dimethoxybenzoesäure-N-(1-((2-acetylaminoethyl)-(3)4 -formyl-5-methyl-1H-pyrrol-2-yl)methyl)-amid

Zu 0,8 g (11 mmol) Dimethylformamid in 20 ml 1,2-Dichlorethan werden bei 10°C 1,69 g (11 mmol)

Phosphoroxychlorid zugetropft. Man rührt 15 Minuten nach, tropft 3,7 g (10 mmol) 3,4-Dimethoxybenzoesäure-N-((1-(2-acetylaminoethyl)-5-methyl-1H-pyrrol-2-yl)methyl)-amid in 20 ml 1,2-Dichlorethan zu und rührt über Nacht bei Raumtemperatur. Der Ansatz wird durch Zutropfen von 4,5 g (33 mmol) Kaliumcarbonat, in 30 ml Wasser gelöst, bei -10°C hydrolysiert. Nach Aufarbeitung analog Beispiel 1 können die beide Isomeren isoliert werden.

1. Verbindung:
3,4-Dimethoxybenzoesäure-N-((1-(2-acetylaminoethyl)-3-formyl-5-methyl-1H-pyrrol-2-yl)methyl)-amid
Schmelzpunkt: 165-167°C (Essigester/Ether)
1H-NMR ($CDCl_3$, TMS), δ(ppm);
1,9 (s, 3H); 2,3 (s, 3H); 3,6 (m, 2H); 3,9 (s, 6H); 4,2 (t, 2H); 4,75 (d, 2H); 6,3 (s, 1H); 6,75 (m, 1H); 6,86 (d, 1H); 7,4 (m, 2H); 8,35 (m, 1H); 9,75 (s, 1H);
2. Verbindung:
3,4-Dimethoxybenzoesäure-N-((1-(2-acetylaminoethyl)-4-formyl-5-methyl-1H-pyrrol-2-yl)methyl)-amid
Schmelzpunkt: 154-156°C (Essigester/Ether)
1H-NMR ($CDCl_3$, TMS), δ(ppm);
2,0 (s, 3H); 2,5 (s, 3H); 3,4 (m, 2H); 3,9 (s, 6H); 4,05 (m, 2H); 4,6 (d, 2H); 6,5 (s, 1H); 6,6 (m, 1H); 6,9 (d, 1H); 7,5 (m, 2H); 7,7 (m, 1H); 9,75 (s, 1H).

## Beispiel 3

### 4-Nitrobenzoesäure-N-((1-(2-acetylaminoethyl-(3)4-formyl -5-methyl-1H-pyrrol-2-yl)methyl)-amid

Analog Beispiel 2 werden 2,5 g (7,26 mmol) 4-Nitrobenzoesäure-N-((1-(2-acetylaminoethyl)-5-methyl-1H-pyrrol-2-yl)methyl)-amid, 1,2 g (7,8 mmol) Phosphoroxychlorid, 0.57 g (7,8 mmol) Dimethylformamid in 40 ml Methylenchlorid umgesetzt.

1. Verbindung:
4-Nitrobenzoesäure-N-((1-(2-acetylaminoethyl-3-formyl-5-methyl-1H-pyrrol-2-yl)methyl)-amid
Schmelzpunkt: 207-210° (Essigester/Ether)
1H-NMR (Polysol, TMS), δ(ppm);
1,9 (s, 3H); 2,3 (s, 3H); 3,4 (m, 2H); 4,1 (t, 2H), 4,8 (d, 2H); 6,25 (s, 1H); 8,1 (m, 1H); 8,15 (d, 2H); 8,25 (d, 2H); 9,2 (t, 1H); 9,95 (s, 1H);
2. Verbindung
4-Nitrobenzoesäure-N-((1-(2-acetylaminoethyl-4-formyl-5-methyl-1H-pyrrol-2-yl)methyl)-amid
Schmelzpunkt: 175-177°C (Essigester/Ether)
1H-NMR (Polysol, TMS), δ(ppm);
1,9 (s, 3H); 2,55 (s, 3H); 3,4 (m, 2H); 4,05 (t, 2H); 4,55 (d, 2H); 6,5 (s, 1H); 8,0 (t, 1H); 8,15 (d, 2H); 8,25 (d, 2H); 9,1 (t, 1 H); 9,8 (s, 1H).

## Beispiel 4

### Essigsäure-N-((1-(2-acetylaminoethyl)-4-formyl-5-methyl-1H-pyrrol-2-yl)methyl)-amid

Zu 3,0 g (21 mmol) Chlormethylendimethylammoniumchlorid in 30 ml 1,2-Dichlorethan werden bei 10°C 4,9 g Essigsäure-N-((1-(2-acetylaminoethyl)-5-methyl-1H-pyrrol-2-yl)methyl)-amid in 70 ml 1,2-Dichlorethan zugetropft. Man rührt 90 min nach, hydrolysiert mit Kaliumcarbonatlösung und arbeitet wie in Beispiel 1 beschrieben auf.
Schmelzpunkt: 162-164°C (Isopropanol/Ligroin)
1H-NMR ($CDCl_3$, TMS), δ(ppm);
1,9 (s, 3H); 1,85 (s, 3H), 2,5 (s, 3H); 3,25 (m, 2H); 3,9 (t, 2H); 4,2 (d, 2H); 6,3 (s, 1H); 8,1 (t, 1H); 8,2 (t, 1H); 9,7 (s, 1H).

## Beispiel 5

### 1H-Pyrrol-1-essigsäure-(2-(acetylaminomethyl)-(3)4-formyl-5-methyl)-methylester

Analog Beispiel 4 werden 6,2 g (27 mmol) 1H-Pyrrol-1-essigsäure-(2-(acetylaminomethyl)-5-methyl)-methylester und 3,9 g (30 mmol) Chlormethylendimethyl-ammoniumchlorid in 100 ml 2-Dichlorethan umgesetzt.

1. Verbindung
1H-Pyrrol-1-essigsäure-(2-(acetylaminomethyl)-3-formyl-5 -methyl)-methylester
Schmelzpunkt: 145-147°C (Essigester)
$^1$H-NMR ($CDCl_3$, TMS), δ(ppm);
1,95 (s, 3H); 2,2 (s, 3H), 3,8 (s, 3H); 4,5 (s, 2H); 4,95 (s, 2H); 6,35 (s, 1H); 7,0 (m, 1H); 9,75 (s, 1H);
2. Verbindung
1H-Pyrrol-1-essigsäure-(2-(acetylaminomethyl)-4-formyl-5-methyl)-methylester
Schmelzpunkt: 135-137°C (Essigester/Ether)
$^1$H-NMR: ($CDCl_3$, TMS) δppm);
2,0 (s, 3H); 2,4 (s, 3H); 3,8 (s, 3H); 4,35 (s, 2H); 4,75 (s, 2H); 6,45 (s, 1H); 6,5 (m, 1H); 9,75 (s, 1H).

Beispiel 6

4-Chlorphenoxyessigsäure-N-((1-(2-acetylaminoethyl)-(3) 4-formyl-5-methyl-1H-pyrrol-2-yl)methyl)-amid

In eine Mischung aus 3,9 ml (50 mmol) Dimethylformamid und 70 ml Methylenchlorid leitet man bei 0°C unter Kühlung und Feuchtigkeitsausschluß und unter Stickstoffüberlagerung 4,9 g (49 mmol) Phosgen innerhalb von 30 Minuten ein. Man rührt 45 Minuten bei 0°C nach und tropft innerhalb von 30 Minuten 13,6 g (37 mmol) 4-Chlorphenoxyessigsäure-N-((1-(2-acetylaminoethyl)-5-methyl-1H-pyrrol -2-yl)methyl)-amid in 30 ml Methylenchlorid zu. Man rührt 20 Minuten bei 0°C, 1 h bei 10-15°C, hydrolysiert nach vollständiger Umsetzung mit Kaliumcarbonatlösung und arbeitet, wie in Beispiel 2 beschrieben, auf.
1. Verbindung
4-Chlorphenoxyessigsäure-N-((1-(2-acetylaminoethyl-3-formyl-5-methyl-1H-pyrrol-2-yl)methyl)-amid
Schmelzpunkt: 177-179°C
$^1$H-NMR (DMSO, TMS), δ(ppm);
1,8 (s, 3H); 2,2 (s, 3H); 3,25 (m, 2H); 3, 95 (t, 2H); 4,5 (s, 2H); 4,6 (d, 2H); 6,2 (s, 1H); 6,95 (dd, 2H); 7,3 (dd, 2H); 8m1 (t, 1H); 8,7 (t, 1H); 9,85 (s, 1H);
2. Verbindung
4-Chlorphenoxyessigsäure-N-((1-(2-acetylaminoethyl-3-formyl-5-methyl-1H-pyrrol-2-yl)methyl)-amid
Schmelzpunkt: 150-152°C
$^1$H-NMR (DMSO, TMS); δ(ppm);
1,8 (s, 3H); 2,5 (s, 3H); 3,25 (m, 2H); 3,95 (t, 2H); 4,3 (d, 2H); 4,55 (s, 2H); 6,3 (s, 1H); 7,0 (dd, 2H), 7,35 (dd, 2H); 8,1 (t, 1H); 8,55 (t, 1H); 9,75 (s, 1H).

Beispiel 7

4-Chlorphenoxyessigsäure-N-(((3)4-formyl-5-methyl-1H-pyrrol-2-yl)methyl)-amid

11 g (39 mmol) 4-Chlorphenoxyessigsäure-N-((5-methyl-1H-pyrrol-2-yl)-methyl)-amid und 5,7 g (45 mmol) Chlormethylendimethylammoniumchlorid werden in 380 ml 1,2-Dichlorethan analog Beispiel 4 umgesetzt.
1. Verbindung
4-Chlorphenoxyessigsäure-N-((3-formyl-5-methyl-1H-pyrrol-2-yl)methyl-amid
Schmelzpunkt: 171-174°C
1H-NMR ($CDCl_3$, TMS), δ(ppm);
2,3 (s, 3H); 4,35 (d, 2H), 4,5 (s, 2H); 6,3 (s, 1H); 6,85 (d, 2H); 6,9 (m, 1H); 7,25 (d, 2H); 9,3 (s, 1H); 11,1 (s, 1H);
2. Verbindung
4-Chlorphenoxyessigsäure-N-((4-formyl-5-methyl-1H-pyrrol-2-yl)methyl)-amid
Schmelzpunkt: 181 - 183°C (Essigester)
1H-NMR (DMSO, TMS), δ(ppm);
2,4 s, 3H); 4,25 (d, 2H); 4,5 (s, 2H); 6,15 (s, 1H); 6,95 (dd, 2H); 7,3 (dd, 2H); 8,4 (s, 1H); 9,75 (s, 1H); 11,2 (s, 1H).

Beispiel 8

Essigsäure-N-(((3)4-formyl-5-methyl-1H-pyrrol-2-yl)methyl)-amid

5,3 g (35 mmol) Essigsäure-N-((5-methyl-1H-pyrrol-2-yl)methyl)-amid und 5,0 g (39 mmol) Chlormethylendimethylammoniumchlorid werden in 120 ml 1,2-Dichlorethan analog Beispiel 4 umgesetzt.

1. Verbindung:
Essigsäure-N-((3-formyl-5-methyl-1H-pyrrol-2-yl)methyl)-amid
Schmelzpunkt: 147 - 150°C
$^1$H-NMR ($CDCl_3$, TMS), $\delta$ (ppm);
1,85 (s, 3H); 2,15 (s, 3H); 4,45 (d, 2H); 6,05 (s, 1H); 8,2 (b, 1H); 9,75 (s, 1H); 11,2 (s, 1H);

2. Verbindung:
Essigsäure-N-((4-formyl-5-methyl-1H-pyrrol-2-yl)methyl)-amid
Schmelzpunkt: 178 - 180°C
$^1$H-NMR ($CDCl_3$, TMS), $\delta$ (ppm);
1,85 (s, 3H); 2,4 (s, 3H); 4,15 (d, 2H); 6,2 (s, 1H); 8,15 (b, 1H); 9,7 (s, 1H); 11,25 (s, 1H).

Die nachfolgenden Beispiele A bis H beschreiben pharmazeutische Zubereitungen

Beispiel A

Emulsionen mit 3 mg Wirkstoff per 5 ml können nach folgender Rezeptur hergestellt werden:

| | |
|---|---|
| Wirsktoff | 0.06 g |
| Neutralöl | q.s. |
| Natriumcarboxymethylzellulose | 0.6 g |
| Polyoxyethylenstearat | q.s. |
| Reinglycerin | 0.6 - 2 g |
| Aromastoffe | q.s. |
| Wasser (entmineralisiert destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden | |
| aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Caliumphosphat | 30 mg |
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propranolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäße Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0.3 mg |
| Natriumchlorid | 2,7 mg |
| Wasser zu Injektionszwecken auf | 1,0 ml |

Zur pharmakologischen Ausprüfung werden die erfindungsgemäßen Verbindungen beispielsweise nach den beiden folgenden Methoden I und II wie folgt geprüft:

I. Nitrit-Hypoxie bei der Maus

In diesem Test wird nach der Methode von Gibson und Blass (J. Neurochem. 27, 37 (1976) bei Mäusen mit $NaNO_2$ (175 mg/kg s. c.) eine cerebrale Hypoxie erzeugt, die zu massiven Verhaltensstörungen der Tiere führt. Bestimmt wird, ob die Haltefähigkeit an einem Drehstab durch Prämedikation mit der Testsubstanz beeinflußt wird. Die Ergebnisse sind in Tabelle 1 angegeben.

Tabelle 1

Prozentuale Umkehr der Störung der Haltefähigkeit nach Verabreichung von 175 mg/kg s. c. $NaNO_2$ und Prämedikation mit den Verbindungen der allgemeinen Formel I.

| Verbindung der allgemeinen Formel I gemäß Beispiel | Dosis (mg/kg) (p.o.) | Prozentuale Umkehr des Hypoxie Effektes |
|---|---|---|
| 1a | 3 | 36 |
| 1b | 3 | 63 |
| 2a | 3 | 71 |
| 2b | 3 | 68 |
| 3a | 3 | 58 |
| 3b | 3 | 55 |
| 4 | 0.03 | 77 |
| 5b | 0.3 | 34 |
| 6a | 0.03 | 69 |
| Piracetam (bekannte Vergleichssubstanz) | 10 | 19 |

Bei den angegebenen Beispielen bedeutet "a" die jeweils erste Verbindung und "b" die jeweils zweite Verbindung des genannten Beispiels.

II. Passive avoidance-Test

Die Testapparatur ist eine Hell-Dunkel-Box mit elektrifizierbarem Gitterboden im dunklen Teil.

55 Minuten nach Verabreichung von Kontroll- und Präparatinjektion werden unerfahrene männliche Mäuse mit Scopolaminhydrobromid (3 mg/kg i. p.) behandelt. 5 Minuten später werden die Mäuse in den hellen Teil der Box gesetzt. Nach dem Überwechseln in den dunklen Teil der Box erhalten sie einen ihnen unangenehmen elektrischen Fußschock. Nach 24 Stunden wird jede Maus einmal in den hellen Teil der Testapparatur gesetzt und die Verweildauer (max. 180 sec.) gemessen. Die mit einer aktiven Dosis eines Präparats und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen. Die Ergebnisse sind in Tabelle 2 angegeben.

Tabelle 2

Prozentuale Abschwächung der durch Scopolamin induzierten Amnesie, erkennbar an einer Verlängerung der Zeit bis zum Betreten des dunklen Teils des Passive-Avoidance Testraumes.

| Verbindung der allgemeinen Formel I gemäß Beispiel | Dosis (mg/kg) (p.o.) | Prozentuale Abschwächung |
|---|---|---|
| 1a | 10 | 34 |
| 1b | 10 | 29 |
| 2b | 3 | 33 |
| 3b | 3 | 25 |
| 4 | 0.3 | 79 |
| 6a | 0.3 | 41 |
| Piracetam (bekannte Vergleichssubstanz) | 30 | 18 |

Bei den angegebenen Beispielen bedeutet "a" die jeweils erste Verbindung und "b" die jeweils zweite Verbindung des genannten Beispiels.

**Patentansprüche**

**1.** 2-(Aminoalkyl)-pyrrolaldehyde der allgemeinen Formel I

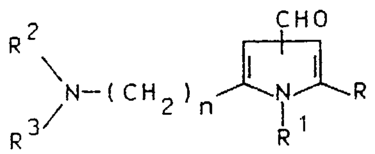

(I)

worin
R: Wasserstoff, Alkyl($C_1$-$C_3$); $R^1$: Wasserstoff, Alkyl($C_1$-$C_5$), Cyanoalkyl($C_1$-$C_4$), Alkoxy($C_1$-$C_4$)carbonyl, Hydroxycarbonylalkyl($C_1$-$C_4$), Alkoxy($C_1$-$C_4$)carbonylalkyl($C_1$-$C_4$), $R^4$($R^5$)N-carbonylalkyl($C_1$-$C_4$), Alkyl-($C_1$-$C_6$)carbonylaminoalkyl($C_1$-$C_4$), Phenyl-carbonylaminoalkyl($C_1$-$C_4$), Phenoxy-alkyl($C_1$-$C_3$)-carbonylaminoalkyl($C_1$-$C_4$), Phenyl, Phenylalkyl($C_1$-$C_4$,); $R^2$, $R^3$; unabhängig voneinander Wasserstoff, Alkyl($C_1$-$C_6$),Alkanoyl($C_1$-$C_6$), Phenylalkyl($C_1$-$C_4$)carbonyl, Phenoxyalkyl($C_1$-$C_3$)carbonyl, Benzoyl, Pyridylcarbonyl, oder zusammen mit dem Stickstoffatom, an das sie gebunden sind, einen 5-gliedrigen Ring bilden;
$R^4$, $R^5$: unabhängig voneinander Wasserstoff, Alkyl($C_1$-$C_4$) oder zusammen mit dem Stickstoffatom, an das sie gebunden sind, einen 5-gliedrigen Ring bilden; wobei die Phenyl-, Phenoxy- oder Benzoylsubstituenten auch durch Halogen, Alkyl($C_1$-$C_4$), Hydroxy, Alkoxy($C_1$-$C_4$), $R^4$($R^5$)N, Mercapto, Alkylmercapto($C_1$-$C_4$), Nitro, Cyano, Hydroxycarbonyl, Alkoxy($C_1$-$C_4$)carbonyl, Alkoxy($C_1$-$C_4$)-carbonylalkyl($C_1$-$C_4$), Formyl, Alkanoyl($C_1$-$C_4$), ein oder mehrfach substituiert sein können;
n: 1, 2 oder 3;
bedeuten, sowie ihre pharmakologisch annehmbaren Säureadditionssalze bei Verbindungen, die eine basische Gruppe enthalten.

**2.** 2-(Aminoalkyl)-pyrrolaldehyde nach Anspruch 1, dadurch gekennzeichnet, daß
R Methyl bedeutet und/oder daß
$R^1$ Wasserstoff, Alkyl($C_1$-$C_6$)carbonylaminoalkyl($C_1$-$C_4$), insbesondere 2-Acetamino-ethyl, Alkoxy($C_1$-$C_4$)-carbonylalkyl($C_1$-$C_4$), insbesondere Methoxy-carbonyl-methyl, bedeutet und/oder daß
$R^2$ Alkanoyl($C_1$-$C_6$), insbesondere Acetyl, Benzoyl, Phenyl-alkyl($C_1$-$C_4$)carbonyl, insbesondere Benzylcarbonyl, Phenoxy-alkyl($C_1$-$C_3$)carbonyl, insbesondere Phenoxy-methylcarbonyl, wobei die Phenylgruppe des Benzoyls oder Phenalkylcarbonyls, insbesondere Benzylcarbonyls, oder die Phenoxygruppe

des Phenoxy-alkyl-($C_1$-$C_3$)carbonyls, insbesondere des Phenoxy-methyl-carbonyls, auch einen oder zwei Substituenten, insbesondere Halogen, Nitro und/oder Alkoxy($C_1$-$C_4$) tragen kann, und/oder $R^3$ Wasserstoff und/oder
n = 1 bedeuten.

**3.** Essigsäure-N-((1-(2-acetylaminoethyl)-4-formyl-5-methyl-1H-pyrrol-2-yl)methyl)-amid.

**4.** 1H-Pyrrol-1-essigsäure-(2-(acetylaminoethyl)-4-formyl-5-methyl)-methylester.

**5.** 4-Chlorphenoxyessigsäure-N-((1-(2-acetylaminoethyl-3-formyl-5-methyl-1H-pyrrol-2-yl)methyl)-amid.

**6.** Verfahren zur Herstellung der 2-(Aminoalkyl)-pyrrolaldehyde der Formel I eines oder mehrerer der Ansprüche 1 bis 5, dadurch gekennzeichnet, daß ein 2-(Aminoalkyl)-pyrrol der Formel II

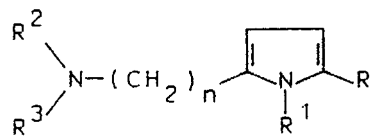

(II)

worin R, $R^1$, $R^2$, $R^3$ und n die in Anspruch 1 und/oder 2 angegebenen Bedeutungen besitzen, in an sich bekannter Weise formyliert wird und, daß gewünschtenfalls ein bei der Formylierung anfallendes Gemisch in an sich bekannter Weise in die Einzelkomponenten aufgetrennt und, falls die hergestellte Verbindung der Formel I eine basische Gruppe enthält, die hergestellte Verbindung gewünschtenfalls in an sich bekannter Weise in ein pharmakologisch annehmbares Säureadditionssalz überführt, oder für den Fall, daß die hergestellte Verbindung als Säureadditionssalz anfällt, aus diesem Säureadditionssalz die freie Verbindung gewünschtenfalls in an sich bekannter Weise isoliert wird.

**7.** Verwendung eines 2-(Aminoalkyl)-pyrrolaldehyds oder eines pharmakologisch annehmbaren Säureadditionsalzes davon, eines oder mehrerer der Ansprüche 1 bis 5 zur Behandlung oder Vorbeugung von Krankheiten beim Menschen, die durch eine Einschränkung der Gehirnfunktion bedingt sind oder zur Behandlung oder Vorbeugung von cerebralen Alterungsvorgängen.

**8.** Verwendung eines 2-(Aminoalkyl)-pyrrolaldehyds oder eines pharmakologisch annehmbaren Säureadditionsalzes davon, eines oder mehrerer der Ansprüche 1 bis 5 zur Herstellung eines Arzneimittels zur Behandlung oder Vorbeugung von Krankheiten beim Menschen, die durch eine Einschränkung der Gehirnfunktion bedingt sind oder zur Behandlung oder Vorbeugung von cerebralen Alterungsvorgängen.

**9.** Pharmazeutisches Präparat, dadurch gekennzeichnet, daß es als Wirkstoff einen oder mehrere 2-(Aminoalkyl)pyrrolaldehyde der allgemeinen Formel I

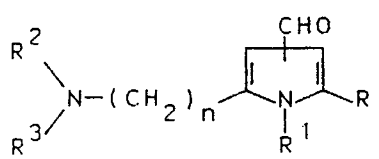

(I)

worin
R: Wasserstoff, Alkyl($C_1$-$C_3$);
$R^1$: Wasserstoff, Alkyl($C_1$-$c_5$), Cyanoalkyl($C_1$-$C_4$), Alkoxy($C_1$-$C_4$)carbonyl, Hydroxycarbonylalkyl($C_1$-$C_4$), Alkoxy($C_1$-$C_4$)carbonylalkyl($C_1$-$C_4$), $R^4(R^5)$N-carbonylalkyl($C_1$-$C_4$), Alkyl($C_1$-$C_6$)carbonylaminoalkyl($C_1$-$C_4$), Phenyl-carbonylaminoalkyl($C_1$-$C_4$,), Phenoxy-alkyl($C_1$-$C_3$)-carbonylaminoalkyl($C_1$-$C_4$,), Phenyl, Phenylalkyl($C_1$-$C_4$,); $R^2$, $R^3$: unabhängig voneinander Wasserstoff, Alkyl($C_1$-$C_6$),Alkanoyl($C_1$-$C_6$), Phenylalkyl($C_1$-$C_4$)carbonyl, Phenoxy-alkyl($C_1$-$C_3$)carbonyl, Benzoyl, Pyridylcarbonyl, oder zusammen mit dem Stickstoffatom, an das sie gebunden sind, einen 5-gliedrigen Ring bilden;
$R^4$, $R^5$: unabhängig voneinander Wasserstoff, Alkyl($C_1$-$C_4$) oder zusammen mit dem Stickstoffatom, an

das sie gebunden sind, einen 5-gliedrigen Ring bilden; wobei die Phenyl-, Phenoxy- oder Benzoylsubstituenten auch durch Halogen, Alkyl($C_1$-$C_4$), Hydroxy, Alkoxy($C_1$-$C_4$), $R^4(R^5)N$, Mercapto, Alkylmercapto($C_1$-$C_4$), Nitro, Cyano, Hydroxycarbonyl, Alkoxy($C_1$-$C_4$)carbonyl, Alkoxy($C_1$-$C_4$)-carbonylalkyl($C_1$-$C_4$), Formyl, Alkanoyl($C_1$-$C_4$), ein oder mehrfach substituiert sein können;
n: 1, 2 oder 3;
bedeuten, oder ein oder mehrere pharmakologisch annehmbare Säureadditionssalze davon zusammen mit einem oder mehreren pharmazeutisch annehmbaren Trägerstoffen und gegebenenfalls einem oder mehreren pharmazeutisch annehmbaren Hilfstoffen und gegebenenfalls einen oder mehrere andere pharmakologische Wirkstoffe enthält.

**10.** Verfahren zur Herstellung eines pharmazeutischen Präparates, dadurch gekennzeichnet, daß ein oder mehrere 2-(Aminoalkyl)-pyrrolaldehyde der allgemeinen Formel I

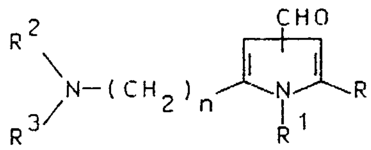

( I )

worin
R: Wasserstoff, Alkyl($C_1$-$C_3$);
$R^1$: Wasserstoff, Alkyl($C_1$-$C_5$), Cyanoalkyl($C_1$-$C_4$), Alkoxy-($C_1$-$C_4$)carbonyl, Hydroxycarbonylalkyl($C_1$-$C_4$), Alkoxy-($C_1$-$C_4$)carbonylalkyl($C_1$-$C_4$), $R^4(R^5)$N-carbonylalkyl($C_1$-$C_4$),Alkyl($C_1$-$C_6$)carbonylaminoalkyl($C_1$-$C_4$), Phenylcarbonylaminoalkyl($C_1$-$C_4$,), Phenoxy-alkyl($C_1$-$C_3$) carbonylaminoalkyl($C_1$-$C_4$,), Phenyl, Phenylalkyl($C_1$-$C_4$,); $R^2$, $R^3$: unabhängig voneinander Wasserstoff, Alkyl($C_1$-$C_6$),Alkanoyl($C_1$-$C_6$), Phenylalkyl($C_1$-$C_4$)carbonyl, Phenoxy-alkyl($C_1$-$C_3$)carbonyl, Benzoyl, Pyridylcarbonyl, oder zusammen mit dem Stickstoffatom, an das sie gebunden sind, einen 5-gliedrigen Ring bilden;
$R^4$, $R^5$: unabhängig voneinander Wasserstoff, Alkyl($C_1$-$C_4$) oder zusammen mit dem Stickstoffatom, an das sie gebunden sind, einen 5-gliedrigen Ring bilden; wobei die Phenyl-, Phenoxy- oder Benzoylsubstituenten auch durch Halogen, Alkyl($C_1$-$C_4$), Hydroxy, Alkoxy($C_1$-$C_4$), $R^4(R^5)N$, Mercapto, Alkylmercapto($C_1$-$C_4$), Nitro, Cyano, Hydroxycarbonyl, Alkoxy($C_1$-$C_4$)carbonyl, Alkoxy($C_1$-$C_4$)-carbonylalkyl($C_1$-$C_4$), Formyl, Alkanoyl($C_1$-$C_4$), ein oder mehrfach substituiert sein können;
n: 1, 2 oder 3;
bedeuten,
oder ein oder mehrere pharmakologisch annehmbare Säureadditionssalze davon als Wirkstoff und ein oder mehrere pharmazeutisch annehmbare Trägerstoffe und gegebenenfalls ein oder mehrere pharmazeutisch annehmbare Hilfstoffe und gegebenenfalls ein oder mehrere andere pharmakologische Wirkstoffe in an sich bekannter Weise in eine zur Verabreichung am Menschen geeignete Form gebracht werden.

**Patentansprüche für folgende Vertragsstaaten: ES, GR**

**1.** Verfahren zur Herstellung von 2-(Aminoalkyl)-pyrrolaldehyden der allgemeinen Formel I

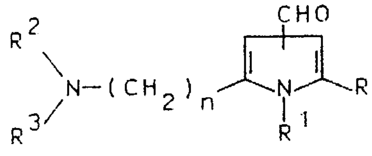

( I )

worin
R: Wasserstoff, Alkyl($C_1$-$C_3$);
$R^1$: Wasserstoff, Alkyl($C_1$-$C_5$), Cyanoalkyl ($C_1$-$C_4$), Alkoxy($C_1$-$C_4$)carbonyl, Hydroxycarbonylalkyl($C_1$-$C_4$), Alkoxy($C_1$-$C_4$)carbonylalkyl(($C_1$-$C_4$), $R^4(R^5)$N-carbonylalkyl($C_1$-$C_4$), Alkyl($C_1$-$C_6$)carbonylaminoalkyl($C_1$-$C_4$), Phenyl-carbonylaminoalkyl($C_1$-$C_4$), Phenoxy-alkyl($C_1$-$C_3$)-carbonylaminoalkyl($C_1$-$C_4$), Phenyl, Phenylalkyl($C_1$-$C_4$,); $R^2$, $R^3$; unabhängig voneinander Wasserstoff, Alkyl($C_1$-$C_6$),Alkanoyl($C_1$-$C_6$), Phenylalkyl($C_1$-$C_4$)carbonyl, Phenoxy-alkyl($C_1$-$C_3$)carbonyl, Benzoyl, Pyridylcarbonyl, oder zusammen

mit dem Stickstoffatom, an das sie gebunden sind, einen 5-gliedrigen Ring bilden;
$R^4$, $R^5$: unabhängig voneinander Wasserstoff, Alkyl($C_1$-$C_4$) oder zusammen mit dem Stickstoffatom, an das sie gebunden sind, einen 5-gliedrigen Ring bilden; wobei die Phenyl-, Phenoxy- oder Benzoylsubstituenten auch durch Halogen, Alkyl($C_1$-$C_4$), Hydroxy, Alkoxy($C_1$-$C_4$), $R^4$($R^5$)N, Mercapto, Alkylmercapto($C_1$-$C_4$), Nitro, Cyano, Hydroxycarbonyl, Alkoxy($C_1$-$C_4$)carbonyl, Alkoxy($C_1$-$C_4$)-carbonylalkyl($C_1$-$C_4$), Formyl, Alkanoyl($C_1$-$C_4$), ein oder mehrfach substituiert sein können;
n: 1, 2 oder 3;
bedeuten, sowie ihrer pharmakologisch annehmbaren Säureadditionssalze bei Verbindungen, die eine basische Gruppe enthalten, dadurch gekennzeichnet, daß ein 2-(Aminoalkyl)-pyrrol der Formel II

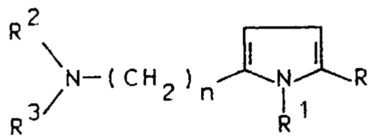

(II)

worin R, $R^1$, $R^2$, $R^3$ und n die bereits angegebene Bedeutungen besitzen, in an sich bekannter Weise formyliert wird und, daß gewünschtenfalls ein bei der Formylierung anfallendes Gemisch in an sich bekannter Weise in die Einzelkomponenten aufgetrennt und, falls die hergestellte Verbindung der Formel I eine basische Gruppe enthält, die hergestellte Verbindung gewünschtenfalls in an sich bekannter Weise in ein pharmakologisch annehmbares Säureadditionssalz überführt, oder für den Fall, daß die hergestellte Verbindung als Säureadditionssalz anfällt, aus diesem Säureadditionssalz die freie Verbindung gewünschtenfalls in an sich bekannter Weise isoliert wird.

**2.** Verfahren nach Anspruch 1, dadurch gekennzeichnet, daß eine Verbindung der Formel II mit R = Methyl eingesetzt wird.

**3.** Verfahren nach Anspruch 1 und/oder 2, dadurch gekennzeichnet, daß eine Verbindung der Formel II eingesetzt wird, bei der $R^1$ Wasserstoff, Alkyl($C_1$-$C_6$)carbonylaminoalkyl($C_1$-$C_4$), insbesondere 2-Acetaminoethyl, Alkoxy($C_1$-$C_4$)-carbonylalkyl($C_1$-$C_4$), insbesondere Methoxy-carbonyl-methyl, bedeutet.

**4.** Verfahren nach einem oder mehreren der Ansprüche 1 bis 3, dadurch gekennzeichnet, daß eine Verbindung der Formel II eingesetzt wird, bei der $R^2$ Alkanoyl($C_1$-$C_6$), insbesondere Acetyl, Benzoyl, Phenylalkyl($C_1$-$C_4$)carbonyl, insbesondere Benzylcarbonyl, Phenoxy-alkyl($C_1$-$C_3$)carbonyl, insbesondere Phenoxy-methyl-carbonyl, wobei die Phenylgruppe des Benzoyls oder Phenalkylcarbonyls, insbesondere Benzylcarbonyls, oder die Phenoxygruppe des Phenoxy-alkyl-($C_1$-$C_3$)carbonyls, insbesondere des Phenoxy-methylcarbonyls, auch einen oder zwei Substituenten, insbesondere Halogen, Nitro und/oder Alkoxy($C_1$-$C_4$) tragen kann, bedeutet.

**5.** Verfahren nach einem oder mehreren der Ansprüche 1 bis 4, dadurch gekennzeichnet, daß eine Verbindung der Formel II eingesetzt wird, bei der $R^3$ Wasserstoff bedeutet.

**6.** Verfahren nach einem oder mehreren der Ansprüche 1 bis 5, dadurch gekennzeichnet, daß eine Verbindung der Formel II eingesetzt wird, bei der n = 1 bedeutet.

**7.** Verfahren nach einem oder mehreren der Ansprüche 1 bis 6, dadurch gekennzeichnet, daß die Ausgangsverbindung II und die Verfahrensbedingungen so ausgewählt werden, daß die Verbindung 1H-Pyrrol-1-essigsäure-(2-(acetylaminoethyl)-4-formyl-5-methyl)-methylester entsteht.

**8.** Verfahren nach einem oder mehreren der Ansprüche 1 bis 6, dadurch gekennzeichnet, daß die Ausgangsverbindung II und die Verfahrensbedingungen so ausgewählt werden, daß die Verbindung 4-Chlorphenoxyessigsäure-N-((1-(2-acetylaminoethyl-3-formyl-5-methyl-1H-pyrrol-2-yl)methyl)-amid entsteht.

**9.** Verfahren nach einem oder mehreren der Ansprüche 1 bis 6, dadurch gekennzeichnet, daß die Ausgangsverbindung II und die Verfahrensbedingungen so ausgewählt werden, daß die Verbindung Essigsäure-N-((1-(2-acetylaminoethyl)-4-formyl-5-methyl-1H-pyrrol-2-yl)methyl)-amid entsteht.

**10.** Verfahren zur Herstellung eines pharmazeutischen Präparates, dadurch gekennzeichnet, daß ein oder mehrere 2-(Aminoalkyl)-pyrrolaldehyde der allgemeinen Formel I

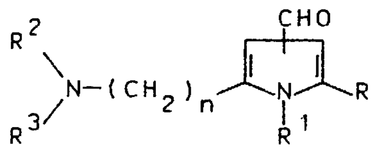

(I)

worin
R: Wasserstoff, Alkyl($C_1$-$C_3$);
$R^1$: Wasserstoff, Alkyl($C_1$-$C_5$), Cyanoalkyl($C_1$-$C_4$), Alkoxy($C_1$-$C_4$)carbonyl, Hydroxycarbonylalkyl($C_1$-$C_4$), Alkoxy($C_1$-$C_4$)carbonylalkyl($C_1$-$C_4$), $R^4$($R^5$)N-carbonylalkyl($C_1$-$C_4$), Alkyl($C_1$-$C_6$)carbonylaminoalkyl($C_1$-$C_4$), Phenyl-carbonylaminoalkyl($C_1$-$C_4$,), Phenoxy-alkyl($C_1$-$C_3$) carbonylaminoalkyl($C_1$-$C_4$,), Phenyl, Phenylalkyl($C_1$-$C_4$,); $R^2$, $R^3$: unabhängig voneinander Wasserstoff, Alkyl($C_1$-$C_6$),Alkanoyl($C_1$-$C_6$), Phenylalkyl($C_1$-$C_4$)carbonyl, Phenoxy-alkyl($C_1$-$C_3$)carbonyl, Benzoyl, Pyridylcarbonyl, oder zusammen mit dem Stickstoffatom, an das sie gebunden sind, einen 5-gliedrigen Ring bilden;
$R^4$, $R^5$: unabhängig voneinander Wasserstoff, Alkyl($C_1$-$C_4$) oder zusammen mit dem Stickstoffatom, an das sie gebunden sind, einen 5-gliedrigen Ring bilden; wobei die Phenyl-, Phenoxy- oder Benzoylsubstituenten auch durch Halogen, Alkyl($C_1$-$C_4$), Hydroxy, Alkoxy($C_1$-$C_4$), $R^4$($R^5$)N, Mercapto, Alkylmercapto($C_1$-$C_4$), Nitro, Cyano, Hydroxycarbonyl, Alkoxy($C_1$-$C_4$)carbonyl, Alkoxy($C_1$-$C_4$)-carbonylalkyl($C_1$-$C_4$), Formyl, Alkanoyl($C_1$-$C_4$), ein oder mehrfach substituiert sein können;
n: 1, 2 oder 3;
bedeuten,
oder ein oder mehrere pharmakologisch annehmbare Säureadditionsalze davon als Wirkstoff und ein oder mehrere pharmazeutisch annehmbare Trägerstoffe und gegebenenfalls ein oder mehrere pharmazeutisch annehmbare Hilfstoffe und gegebenenfalls ein oder mehrere andere pharmakologische Wirkstoffe in an sich bekannter Weise in eine zur Verabreichung am Menschen geeignete Form gebracht werden.

Europäisches Patentamt

# EUROPÄISCHER RECHERCHENBERICHT

Nummer der Anmeldung

**EP 91 10 9826**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 124 067 (CASSELLA AKTIENGESELLSCHAFT)<br>* Seiten 1 - 3, Zeile 1 * * Seite 13 - Zeile 20 *<br>– – – | 1,7 | C 07 D 207/335<br>C 07 D 207/337<br>A 61 K 31/40 |
| A | DE-A-1 720 020 (WHITEFIN HOLDING S.A.)<br>* das ganze Dokument *<br>– – – | 1,7 | |
| A | FR-M-6 239 (ENDO LABORATORIES,INC.)<br>* Seite 1, Spalte 1 - Spalte 2, Absatz 3 *<br>– – – – – | 1,7 | |

| RECHERCHIERTE SACHGEBIETE (Int. Cl.5) |
|---|
| C 07 D 207/00 |

Der vorliegende Recherchenbericht wurde für alle Patentansprüche erstellt

| Recherchenort | Abschlußdatum der Recherche | Prüfer |
|---|---|---|
| Berlin | 08 Oktober 91 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument